# EUROPEAN PATENT SPECIFICATION

(11) **EP 4 386 369 B1**
(45) Date of publication and mention of the grant of the patent: **18.11.2026**
(21) Application number: 22214102.0
(22) Date of filing: 16.12.2022
(51) Int. Cl.: G01N 27/414, G01N 33/487, G01R 31/26

(54) **AN AUTOMATED CALIBRATION LOOP FOR A BIO-FET**
AUTOMATISCHE KALIBRIERSCHLEIFE FÜR EINEN BIO-FET
BOUCLE D'ÉTALONNAGE AUTOMATIQUE POUR BIO-FET

(43) Date of publication of application: 19.06.2024
(73) Proprietor: Imec VZW, 3001 Leuven (BE); Katholieke Universiteit Leuven, 3000 Leuven (BE)
(72) Inventor: Das, Auro, 3000 Leuven (BE); Van Helleputte, Nick, 3060 Korbeek-Dijle (BE)
(74) Representative: Roth, Sebastian

(56) References cited:
- CN-A- 104 614 404
- JP-A- S60 225 056
- US-A1- 2002 117 693

## Description

### TECHNICAL FIELD

The present disclosure relates to biosensor devices, in particular, to biosensor devices based on biosensor field effect transistors (bio-FETs). The disclosure proposes a biosensor device comprising a bio-FET and a readout circuit, which is connected to the bio-FET and is configured to readout the bio-FET and to automatically calibrate the bio-FET.

### BACKGROUND OF THE INVENTION

FET-based biosensor devices are becoming popular, due to the ease of integration of their readout interfaces with CMOS technology. The biosensor devices can be used in a wide range of applications, including electrochemical sensing, DNA sequencing, protein detection etc. The biosensor devices include one or more bio-FETs, for example, nanopore FETs.

For some applications, like DNA or protein detection using nanopore FETs, the signal is very fast (about 100 ns per base). Hence, a readout circuit for the nanopore FET needs to be very fast as well (e.g., it should have a bandwidth of 10 MHz or more), in order to be able to correctly capture the sensing signal. Moreover, due to non-idealities in the fabrication process and due to effects of external factors, the bio-FETs may suffer from issues such as offset and drift of their bias current. Several compensation techniques, such as calibration methods, are conventionally be used to address these issues.

However, some of the conventional compensation techniques are applicable only where the signal bandwidth is quite low, e.g., can be used for ion-sensitive field-effect transistor (ISFET) based biosensor devices. These compensation techniques cannot be applied to bio-FETs with higher bandwidth requirements. Some other conventional compensation techniques require access to the gate of the FET, which is not possible for many bio-FETs, for example, for nanopore FETs, as they are liquid-gated.

Conventional readout circuits for bio-FETs can typically achieve a bandwidth of up to 1 MHz. Hence, they are not applicable for faster applications such as DNA or protein analysis, for which the bandwidth requirement is higher than 1 MHz. Moreover, conventional wideband readout circuits are not fully integrable into an integrated circuits (IC), and hence cannot be used for biosensor devices that include large arrays of many bio-FETs.

CN 104614404 A discloses an ion sensitive field effect transistor sensor and reading circuit thereof
US 2002117693 A1 discloses an odor sensor with organic transistor circuitry.

JPS60 225056 A discloses an ion monitor apparatus.

### SUMMARY OF THE INVENTION

In view of the above, the present disclosure aims for improved compensation techniques. An objective is to provide a circuit and a method that can be used to calibrate a bio-FET. In particular, an objective is to provide a readout circuit for the bio-FET, which is configured to automatically calibrate the bio-FET. An objective thereby is to handle non-idealities of the bio-FET, for instance, to compensate an offset or drift in the bias current of the bio-FET. This should even be possible for applications, which have a high bandwidth requirement of, for example, 1 MHz or more. Another objective is that the readout circuit can be used for large arrays including multiple bio-FETs.

These and other objectives are achieved by the solutions of this disclosure provided in the independent claims. Advantageous implementations are described in the dependent claims.

A first aspect of this disclosure provides a biosensor device as defined in claim 1, the biosensor comprising one or more bio-FETs; and one or more readout circuits, wherein each readout circuit is connected to a source node of one bio-FET of the bio-FETs and is configured to: generate a readout signal based on a bias current of the bio-FET; generate a calibration signal based on a current difference between the bias current of the bio-FET and a reference current; and control a bias of the source node of the bio-FET according to the calibration signal.

By generating the readout signal, the readout circuit is configured to read out a signal that is generated by the bio-FET during a sensing operation. By generating the calibration signal, and by controlling the bias of the source node according to the calibration signal, the readout circuit is configured to calibrate the bio-FET. In particular, the control of the bias of the source node may be done by supplying a control signal to the source node. In this way, the readout circuit may compensate an offset and/or a drift of the bias current of the bio-FET. Thus, the readout circuit can be used for both reading out and automatically calibrating the bio-FET. The readout circuit of the first aspect is compatible with signal bandwidth requirements of 1 MHz or more, and can be fully integrated into an IC.

The readout signal is generated based on high-frequency changes of the bias current of the bio-FET; and the calibration signal is generated based on low-frequency changes and/or a drift of the bias current of the bio-FET.

Thus, faster (high-frequent) signal changes, which are produced when the bio-FET is sensing, can be correctly reflected in the readout signal. At the same time, the calibration signal can be used to compensate slower (low-frequent) changes and/or a slow drift of the bias current of the bio-FET. The frequency value of the faster signal changes can be adjusted according to a cutoff frequency set by the optional filter circuit described below, and can, for example, vary in the range of 1-1000 Hz (e.g., for DNA/protein sequencing) or in the range of 1-10000 Hz. That is, any signal faster than the cutoff frequency of the filter circuit may be transferred to the output. Similarly, any signal slower than the cutoff frequency of the filter circuit may not be transferred to the output, but may determine the calibration signal.

In an implementation of the biosensor device, the readout circuit comprises a current comparator circuit configured to generate the calibration signal based on the bias current of the bio-FET and the reference current.

The reference current may be provided to the readout circuit, or may be generated by the readout circuit. The calibration of the bio-FET based on the difference of the two currents is fast and can adjust automatically.

The readout circuit comprises a filter circuit; the filter circuit is configured to act as a low-pass filter for a calibration signal path of the readout circuit, to prevent that high-frequency changes of the bias current of the bio-FET are used for generating the calibration signal; and the filter circuit is configured to act as a high-pass filter for a readout signal path of the readout circuit, to prevent that low-frequency changes and/or a drift of the bias current of the bio-FET are used for generating the readout signal.

The filter circuit is thus arranged as a low-pass filter in the calibration signal path (also referred to as feedback path). The filter circuit may be part of the current comparators circuit. The filter circuit behaves as a high-pass filter, which rejects an offset and/or drift of the bias current (which are at lower frequency) in the readout signal path to the output of the readout circuit. Moreover, the readout circuit may naturally behave like a low-pass filter (in the readout signal path to the output). Hence, the combined effect may be that of a band-pass filter, which allows a signal only within a specific range of frequencies to pass to the output.

In an implementation of the biosensor device, the current comparator circuit comprises a first amplifier and a series-connected transistor pair, wherein a mid-point of the series-connected transistor pair is connected to an inverting input of the first amplifier; a reference voltage is connected to a non-inverting input of the first amplifier; the transistor pair is configured to provide the current difference between the bias current of the bio-FET and the reference current as a voltage difference compared to the reference voltage to the inverting input of the first amplifier; and the first amplifier is configured to provide the calibration signal at its output.

The series-connected transistor pair may be an inverter.

In an implementation, the biosensor device further comprises a capacitor connected to the inverting input of the first amplifier and the output of the first amplifier; and a pseudo-resistor connected between the mid-point of the series-connected transistor pair and the inverting input of the first amplifier; wherein the capacitor, the pseudo-resistor, and the first amplifier form the filter circuit.

Accordingly, the filter circuit may be integrated with the current comparator circuit. Generally, as described above, the readout circuit provides the filter function and the current comparator function. These two functions may be integrated into one physical circuitry, namely the current comparator circuit. However, it may be possible two separate the two functions into two separate circuits.

In an implementation of the biosensor device, the readout circuit comprises a first current mirror circuit, which includes a first transistor and a second transistor and is configured to mirror the reference current from the first transistor to the second transistor; the readout circuit comprises a second current mirror circuit, which includes a third transistor and a fourth transistor and is configured to mirror the bias current of the bio-FET from the third transistor to the fourth transistor; and he series-connected transistor pair is formed by the second transistor and the fourth transistor.

In an implementation of the biosensor device, the readout circuit further comprises an output transistor; the third transistor and the fourth transistor are connected to the output transistor; and the output transistor is configured to generate the readout signal based on the bias current of the bio-FET.

The output transistor is connected to both the third transistor and the fourth transistor, which means it gets the mirrored current from the third transistor and the fourth transistor, and the fourth transistor gets the current mirror from the third transistor.

In an implementation of the biosensor device, the readout circuit further comprises a control circuit configured to control the bias of the source node of the bio-FET according to the calibration signal provided by the current comparator circuit.

The control circuit may output a control signal, which is produced based on the calibration signal. The control signal may be provided to the source node of the bio-FET, in order to calibrate the bio-FET. Notably, it is possible that the calibration signal is the same as the control signal. The control of the source node bias may change the bias current in the bio-FET, and may thus compensate an offset and/or drift of the bias current.

In an implementation of the biosensor device, the control circuit comprises a second amplifier; the output of the first amplifier is connected to a non-inverting input of the second amplifier; and the source node of the bio-FET is connected to an inverting input of the second amplifier.

In an implementation of the biosensor device, the readout circuit further comprises an input transistor; the input transistor is connected between the source node of the bio-FET and the third transistor of the second current mirror circuit; and the gate of the input transistor is connected to an output of the second amplifier.

In an implementation of the biosensor device, the one or more bio-FETs each comprise a liquid gate.

In an implementation of the biosensor device, the one or more bio-FETs comprise one or more nanopore FETs.

In an implementation of the biosensor device, the biosensor device comprises an array of bio-FETs and an array of readout circuits connected to the array of bio-FETs.

As the readout circuit can be fabricated with CMOS technology and can be integrated into an IC, it is also compatible with biosensor devices having a large number of bio-FETs.

In an implementation of the biosensor device, the array of readout circuits comprises one or more groups of readout circuit; and each group of readout circuits is connected to one or more multiplexers.

A second aspect of this disclosure provides a method for calibrating a biosensor device comprising one or more bio-FETs and one or more readout circuits as defined in claim 14.

The method of the second aspect is a calibration method for a bio-FET. The method of the second aspect achieves the same advantages as described above for the biosensor device of the first aspect, and may be extended by respective implementations corresponding to those described above for the biosensor device of the first aspect. In particular, any feature of the first aspect may be correspondingly implemented in the second aspect.

In summary of the above aspects and implementations, the disclosure proposes the readout circuit, which has a current-conveyor architecture with an automated calibration signal path. In the calibration signal path, the readout circuit senses an error (with respect to the reference current) in the bias current of the bio-FET, and controls the source node of the bio-FET to bias it, for instance, with a desired current magnitude.

The readout circuit is designed such that slow-moving and DC artifacts such as offset and drift are attenuated, while fast-moving signals, which are of interest, are passed to the output. The calibration signal path comprises the low-pass filter, which behaves as a high-pass filter in the readout signal path. The calibration signal path may specifically be configured according to a desired pass-band corner frequency.

### BRIEF DESCRIPTION OF THE DRAWINGS

The above described aspects and implementations are further explained in the following description of embodiments with respect to the following enclosed drawings:
- FIG. 1: shows a biosensor device according to an embodiment of this disclosure.
- FIG. 2: shows an exemplary biosensor device according to an embodiment of this disclosure.
- FIG. 3: illustrates a compensation of an offset and drift in the readout signal of a bio-FET in a biosensor device according to an embodiment of this disclosure.
- FIG. 4: shows examples for (a) a first amplifier, (b) a second amplifier, and (c) a pseudo-resistor, of a biosensor device according to an embodiment of this disclosure.
- FIG. 5: shows a biosensor device according to an embodiment of this disclosure with an array of bio-FETs and an array of readout circuits.
- FIG. 6: shows a calibration method according to an embodiment of this disclosure for calibrating a bio-FET of a biosensor device.

### DETAILED DESCRIPTION OF EMBODIMENTS OF THE INVENTION

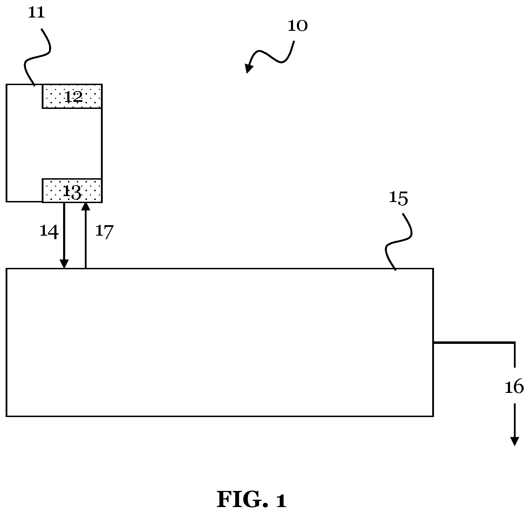

FIG. 1 shows a biosensor device 10 according to an embodiment of this disclosure. The biosensor device 10 is a FET-based biosensor device, which comprises readout circuitry that is compatible with CMOS technology and can be integrated into an IC. The biosensor device 10 can be used for electrochemical sensing, DNA sequencing, protein detection, or similar applications.

The biosensor device 10 comprises at least one bio-FET 11 and at least one readout circuit 15 (one of each is shown in FIG. 1), wherein the readout circuit 15 is connected to a source node 13 of the bio-FET 11. The bio-FET 11 may be a liquid-gated bio-FET, i.e., it may comprise a liquid gate. The bio-FET 11 may, for instance, be a nanopore FET.

The bio-FET 11 comprises a drain node 12 and the source node 13, and may have a channel arranged between the drain node 12 and the source node 13. A surface above the channel may be exposed to a liquid in case of a liquid gate. The bio-FET 11 may be gated by changes in the surface potential of the channel surface, wherein such changes may be induced, for instance, by charged molecules in the liquid gate. These charged molecules may bind to the sensor surface. The charged molecules can influence a charge distribution of a semiconductor material below the channel surface (may be a dielectric surface), and may result in a change of the bias current flowing between the drain node 12 and the source node 13 of the bio-FET 11.

A nanopore FET may be a nanopore sensor including a FET (e.g., a bio-FET). A nanopore sensor is a device that can have a sensing region inside a nanopore or incorporated in another location in the device. The nanopore may be arranged outside of the channel of the FET. In one embodiment, the FET is embedded in the nanopore. In another embodiment, the sensor is a sensing layer (which may be a conductive layer at least embracing and/or wrapping the nanopore) and is electronically coupled to the FET. The nanopore is nanoscale, i.e., it is less than 1 µm in a cross-sectional diameter. For a circular cross-section of the nanopore, that may amount to a diameter of less than 1000 nm.

A nanopore FET may accordingly combine a nanopore with a bio-FET, wherein the nanopore may be a nanoribbon, a nanotube, or a nanowire. The nanopore may also be embedded within the channel of the bio-FET. The nanopore FET may be used to sense single molecules with the bio-FET, wherein the single molecules translocate through the nanopore. The nanopore FET is particularly suited for DNA sequencing or other applications at MHz bandwidths. The advantages of a nanopore FET over a biological nanopore are its mechanical robustness, higher throughput, and higher signal to noise ratio.

The readout circuit 15 is configured to readout a signal generated by the bio-FET 11 during a sensing operation (e.g., the bio-FET 11 produces a switching of the bias current due to the presence of charged molecules, as explained above), and is additionally configured to calibrate the bio-FET 11, as will be explained below. The biosensor device 10 may also comprise multiple bio-FETs 11 and multiple readout circuits 15, for instance, arranged in a sensing array. In this case, each readout circuit 15 is configured as shown in FIG. 1, and is connected to the source node 13 of one of the bio-FETs.

The readout circuit 15, or each readout circuit 15 in the case of multiple readout circuits 15, is configured to generate a readout signal 16 based on the bias current 14 of the bio-FET 11, to which it is connected. In this way, the readout circuit 15 can readout the signal produced according to the sensing of the bio-FET 11. The readout signal 16 is generated based on high-frequency changes of the bias current 14 of the bio-FET 11, which may be caused by the bio-FET 11 sensing charged molecules at its sensor surface.

Further, the readout circuit 15 is configured to generate a calibration signal, and to control a bias of the source node 13 of the bio-FET 11 according to the calibration signal, e.g., by providing a control signal 17 that depends on the calibration signal to the source node 13. In this way, the readout circuit 15 can calibrate the bio-FET 11. The readout circuit 15 is configured to generate the calibration signal based on a current difference between the detected bias current 14 of the bio-FET 11 and a reference current. The reference current may be provided externally to the readout circuit 15, or may be generated by the readout circuit 15. The calibration signal is generated based on low-frequency changes of the bias current 14 and/or based on a drift of the bias current 14 of the bio-FET 11.

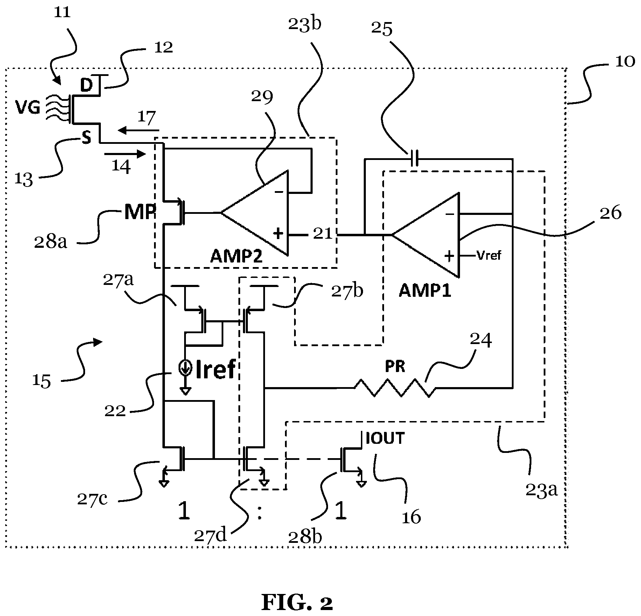

FIG. 2 shows a specific biosensor device 10 according to an embodiment of this disclosure, which builds on the embodiment shown in FIG. 1. Same elements in FIG. 1 and FIG. 2 share the same reference signs, and may be implemented likewise. In particular, FIG. 2 shows an exemplary implementation of the readout circuit 15 connected to the bio-FET 11. As mentioned above, the biosensor device 10 can comprise multiple such readout circuits 15, wherein each is connected to one of multiple bio-FETs 11.

The exemplary readout circuit 15 shown in FIG. 2 comprises various sub-circuits. In particular, the readout circuit 15 comprises a current comparator circuit 23a, which is configured to generate the calibration signal 21 based on the bias current 14 of the bio-FET 11 and based on the reference current 22. Further, the biosensor device 10 comprises a control circuit 23b, which is configured to control the bias of the source node 13 of the bio-FET 11 according to the calibration signal 21 that is provided by the current comparator circuit 23. The readout circuit 15 also comprises a first current mirror circuit, which is formed by a first transistor 27a and a second transistor 27b and is configured to mirror the reference current 22 from the first transistor 27a to the second transistor 27b. The readout circuit 15 further comprises second current mirror circuit, which is formed by a third transistor 27c and a fourth transistor 27d and is configured to mirror the bias current 14 of the bio-FET 11 from the third transistor 27c to the fourth transistor 27d.

FIG. 2 shows exemplary implementations of the above-described sub-circuits, which will be explained in the following. Notably, electrical components of the readout circuit 15 may belong to more than one sub-circuit.

The exemplary current comparator circuit 23a comprises a first amplifier 26 and a series-connected transistor pair formed by the second transistor 27b and the fourth transistor 27d. A mid-point of the series-connected transistor pair, i.e., the point in the middle between the second transistor 27b and the fourth transistor 27d, is connected to the inverting input of the first amplifier 26. A reference voltage (Vref) is connected to the non-inverting input of the first amplifier 26. The series-connected transistor pair is configured to generate the current difference between the bias current 14 and the reference current 22, and to provide the generated current difference as a voltage difference between the voltage at the mid-point of the series-connected transistor pair and the reference voltage to the first amplifier 26. Based on these inputs to the inverting input and the non-inverting input, the first amplifier 26 is configured to provide the calibration signal 21 at its output, in particular, to provide it to the control circuit 23b.

Notably, as shown in FIG. 2, the pseudo-resistor 24 is connected between the mid-point of the series-connected transistor pair and the inverting input of the first amplifier 26. However, the pseudo-resistor 24 plays functionally no role in the current comparison, but is functionally used for providing a filter-circuit.

In particular, the current comparator circuit 23a also comprises a capacitor 25, which is connected to the inverting input of the first amplifier 26 and to the output of the first amplifier 26, respectively. Together with the pseudo-resistor 25 and the first amplifier 26, the capacitor 25 forms a filter circuit. This filter circuit is designed to act as a low-pass filter in the calibration signal path of the readout circuit 15. In this way, the filter circuit can prevent that high-frequency changes of the bias current 14 of the bio-FET 11 are used for generating the calibration signal 21. The pseudo-resistor may be designed to achieve a desired pole frequency and pass-band corner frequency.

As described above, the readout circuit 15 provides the filter function and the current comparator function. In the embodiment shown in FIG. 2, these two functions are integrated into the current comparator circuit 23a. However, it may be possible two separate these two functions into two separate circuits.

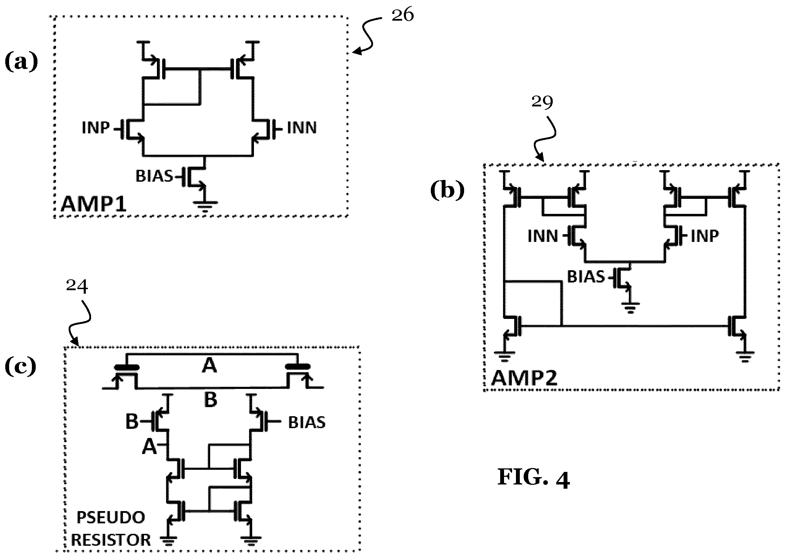

Exemplary implementations of the first amplifier 26 and of the pseudo-resistor are, respectively, shown in FIG. 4(a) and in FIG. 4(c).

The exemplary control circuit 23b comprises a second amplifier 29. The output of the first amplifier 26 is connected to the non-inverting input of the second amplifier 29. The source node 13 of the bio-FET 11 is connected to the inverting input of the second amplifier 29. The control circuit 23b further comprises an input transistor 28a, which is connected between the source node 13 and the third transistor 27c. The gate of the input transistor 28a is connected to the output of the second amplifier 29.

An exemplary implementation of the second amplifier 29 is shown in FIG. 4(b).

The readout circuit 15 further comprises an output transistor 28b, which is connected to the third transistor 27c and to the fourth transistor 27d. The output transistor 28b is configured to generate the readout signal 16. The filter circuit in the current comparator circuit 23b is further designed to act as a high-pass filter for the readout signal path of the readout circuit 15. In this way, the filter circuit can prevent that low-frequency changes of the bias current 14 and/or a drift of the bias current 14 of the bio-FET 11 are used for generating the readout signal 16.

The exemplary readout circuit 15 of FIG. 2 combines a current conveyor (the readout signal path, via the second current mirror circuit and the output transistor 28b, and the control circuit 23b) with a feedback calibration loop (the calibration signal path, via the current comparator circuit 23a and the control circuit 23b; note that the control circuit 23b may be part of both the readout signal path and the calibration signal path). This allows high-frequency signals to be copied out by the second current mirror circuit, while blocking a DC offset and low frequency drift. In addition, slow changes of the bias current 14 of the bio-FET 11 can be compensated, particularly, by biasing the source node 13 of the bio-FET 11 according to the calibration signal 21.

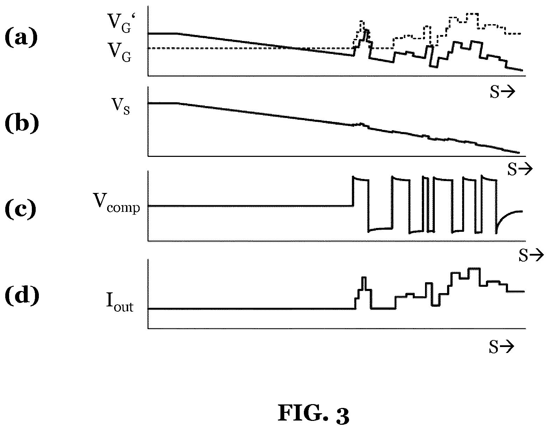

FIG. 3 illustrates such compensation of an offset and drift in the bias current of the bio-FET 11. FIG. 3(a) shows the voltage of the gate of the bio-FET 11, in particular, shows the gate voltage V_{G}' which slowly drifts and exhibits higher-frequent changes due to the sensing of the bio-FET. The drift is reflected in the bias current. The gate voltage without the drift would be as indicated by V_{G}. The drift of V_{G}' can be compensated by biasing the source node 13 of the bio-FET 11, i.e., by adapting the source voltage Vs at the source node 13, which is shown in FIG. 3(b). In the readout signal 16 of the readout circuit 15, in this case in the output current Iₒᵤₜ shown in FIG. 3(d), the drift is suppressed. FIG. 3(c) shows the voltage at the mid-point of the series-connected transistor pair. This voltage is maintained at the reference voltage (connected to the non-inverting input of the first amplifier 26) when the bias current in the bio-FET 11 is the same as the reference current 22. Due to changes in the bias current of the bio-FET 11, there may arise a deviation in the voltage at the mid-point compared to the reference voltage, as the balance is lost. The calibration signal 21 is generated in this case, and may be used to bring the balance back at this point.

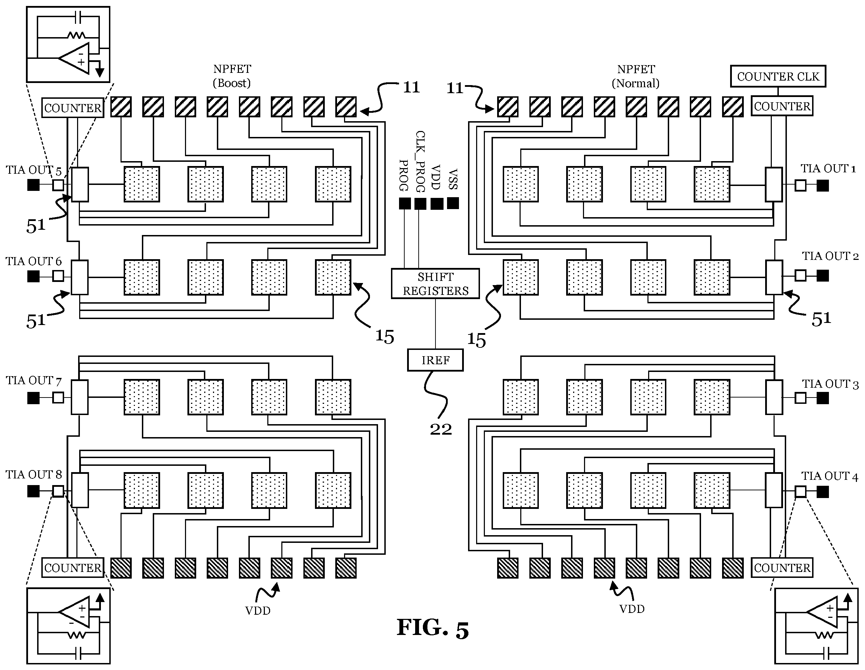

FIG. 5 shows a biosensor device 10, which comprises multiple bio-FETs 11 and multiple readout circuits 15, as already mentioned above. The bio-FETs 11 may be arranged in an array of bio-FETs 11, and the readout circuits 15 may be arranged in an array of readout circuits 15. The array of readout circuits 15 is connected to the array of bio-FETs 11. For example, the array of bio-FETs 11 may comprise sixteen nanopore FETs. Multiple readout circuits 15 of the array, which are grouped together, may be connected to at least one multiplexer 51. In this way, the corresponding bio-FETs 11 can be readout in a multiplexed manner, and the biosensor device 10 can be designed more compactly. The readout circuits 15 may each perform an automatic calibration of their connected bio-FETs 11, especially to handle offsets and drift in the respective bias currents. With the exemplary biosensor device 10, a bandwidth of 10 MHz or more may be achieved.

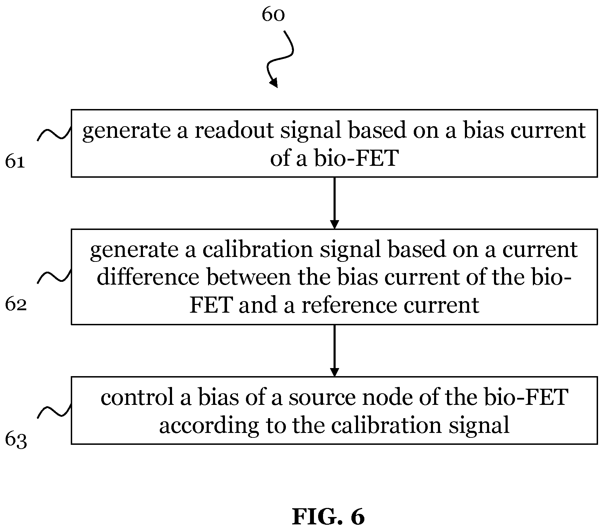

FIG. 6 shows a flow-diagram of a calibration method 60 according to an embodiment of this disclosure. The calibration method 60 may be used to calibrate a bio-FET 11, which is connected to a readout circuit 15 with its source node 13, as shown e.g. in FIG. 1. The calibration method 60 may also be used to calibrate multiple bio-FETs 11 by using multiple readout circuits 15, for instance, as in the biosensor device 10 shown in FIG. 5.

According to the flow-diagram of FIG. 6, the method 60 comprises a step 61 of generating a readout signal 16 based on a bias current 14 of the bio-FET 11. The readout signal 16 is generated by the readout circuit 15 as explained above. The method 60 further comprises a step 62 of generating a calibration signal 21 based on a current difference between the bias current 14 of the bio-FET 11 and a reference current 22, and a step 63 of controlling a bias of the source node 13 of the bio-FET 11 according to the calibration signal 21.

This automated calibration method 60 does not require any digital to analog converters (DACs), and also no separate calibration step before start of recording readout signals from the bio-FET(s) 11. The readout circuit can be operated to provide the readout signal 16 and at the same time perform the calibration of the bio-FET 11. This reduces the design overhead required for large arrays of bio-FETs.

Possible applications for the biosensor device 10 of embodiments of this disclosure are DNA/protein detection and sequencing using nanopore FETs as the bio-FETs 11, or applications where a large signal bandwidth is needed.

In the claims as well as in the description of this disclosure, the word "comprising" does not exclude other elements or steps and the indefinite article "a" or "an" does not exclude a plurality. A single element may fulfill the functions of several entities or items recited in the claims. The mere fact that certain measures are recited in the mutual different dependent claims does not indicate that a combination of these measures cannot be used in an advantageous implementation. The scope of the present invention is defined by the appended claims.

## Claims

1. A biosensor device (10) comprising
one or more biosensor field effect transistors, bio-FETs (11); and
one or more readout circuits (15), wherein each readout circuit (15) is connected to a source node (13) of one bio-FET (11) of the bio-FETs (11) and is configured to
generate a readout signal (16) based on a bias current (14) of the bio-FET (11);
generate a calibration signal (21) based on a current difference between the bias current (14) of the bio-FET (11) and a reference current (22); and
control (17) a bias of the source node (13) of the bio-FET (11) according to the calibration signal (21), **characterised in that**
the readout signal (16) is generated based on high-frequency changes of the bias current (14) of the bio-FET; and
the calibration signal (21) is generated based on low-frequency changes and/or a drift of the bias current (14) of the bio-FET (11),
wherein the readout circuit (15) comprises a filter circuit (24, 25, 26);
the filter circuit (24, 25, 26) is configured to act as a low-pass filter for a calibration signal path of the readout circuit (15), to prevent that high-frequency changes of the bias current (14) of the bio-FET (11) are used for generating the calibration signal (21); and
the filter circuit (24, 25, 26) is configured to act as a high-pass filter for a readout signal path of the readout circuit (15), to prevent that low-frequency changes and/or a drift of the bias current (14) of the bio-FET (11) are used for generating the readout signal (16).

2. The biosensor device (10) according to claim 1, wherein
the readout circuit (15) comprises a current comparator circuit (23a) configured to generate the calibration signal (21) based on the bias current (14) of the bio-FET (11) and the reference current (22).

3. The biosensor device (10) according to claim 2, wherein
the current comparator circuit (23a) comprises a first amplifier (26) and a series-connected transistor pair (27b, 27d), wherein a mid-point of the series-connected transistor pair (27b, 27d) is connected to an inverting input of the first amplifier (26);
a reference voltage (Vref) is connected to a non-inverting input of the first amplifier (26);
the transistor pair (27b, 27d) is configured to provide the current difference between the bias current (14) of the bio-FET (11) and the reference current (22) as a voltage difference compared to the reference voltage (Vref) to the inverting input of the first amplifier (26); and
the first amplifier (26) is configured to provide the calibration signal (21) at its output.

4. The biosensor device (10) according to claim 3, further comprising
a capacitor (25) connected to the inverting input of the first amplifier (26) and the output of the first amplifier (26); and
a pseudo-resistor (24) connected between the mid-point of the series-connected transistor pair (27b, 27d) and the inverting input of the first amplifier (26);
wherein the capacitor (25), the pseudo-resistor (24), and the first amplifier (26) form the filter circuit (24, 25, 26).

5. The biosensor device (10) according to claim 3 or 4, wherein
the readout circuit (15) comprises a first current mirror circuit, which includes a first transistor (27a) and a second transistor (27b) and is configured to mirror the reference current (22) from the first transistor (27a) to the second transistor (27b);
the readout circuit (15) comprises a second current mirror circuit, which includes a third transistor (27c) and a fourth transistor (27d) and is configured to mirror the bias current (14) of the bio-FET (11) from the third transistor (27c) to the fourth transistor (27d); and
the series-connected transistor pair (27b, 27d) is formed by the second transistor (27b) and the fourth transistor (27d).

6. The biosensor device (10) according to claim 5, wherein
the readout circuit (15) further comprises an output transistor (28);
the third transistor (27c) and the fourth transistor (27d) are connected to the output transistor (28); and
the output transistor (28) is configured to generate the readout signal (16) based on the bias current (14) of the bio-FET (11).

7. The biosensor device (10) according to one of the claims 2 to 6, wherein
the readout circuit (15) further comprises a control circuit (23b) configured to control the bias of the source node (13) of the bio-FET (11) according to the calibration signal (21) provided by the current comparator circuit (23a).

8. The biosensor device (10) according to the claims 3 and 7, wherein
the control circuit (23b) comprises a second amplifier (29);
the output of the first amplifier (26) is connected to a non-inverting input of the second amplifier (29); and
the source node (13) of the bio-FET (11) is connected to an inverting input of the second amplifier (29).

9. The biosensor device (10) according to claim 8, wherein
the readout circuit (15) further comprises an input transistor (28a);
the input transistor (28a) is connected between the source node (13) of the bio-FET (11) and the third transistor (27c) of the second current mirror circuit; and
the gate of the input transistor is connected to an output of the second amplifier (29).

10. The biosensor device (10) according to one of the claims 1 to 9, wherein the one or more bio-FETs (11) each comprise a liquid gate.

11. The biosensor device (10) according to one of the claims 1 to 10, wherein the one or more bio-FETs (11) comprise one or more nanopore FETs.

12. The biosensor device (10) according to one of the claims 1 to 11, wherein the biosensor device (10) comprises
an array of bio-FETs (11) and an array of readout circuits (15) connected to the array of bio-FETs (11).

13. The biosensor device (10) according to claim 12, wherein
the array of readout circuits (15) comprises one or more groups of readout circuits (15); and
each group of readout circuits (15) is connected to one or more multiplexers (51).

14. A method for calibrating a biosensor device (10) comprising
one or more biosensor field effect transistors, bio-FETs (11); and
one or more readout circuits (15), wherein each readout circuit (15) is connected to a source node (13) of one bio-FET (11) of the bio-FETs (11), and wherein the method comprises:
generating, with the readout circuit (15), a readout signal (16) based on a bias current (14) of the bio-FET (11);
generating a calibration signal (21) based on a current difference between the bias current (14) of the bio-FET (11) and a reference current (22); and
controlling (17) a bias of the source node (13) of the bio-FET (11) according to the calibration signal (21); **characterised in that**
the readout signal (16) is generated based on high-frequency changes of the bias current (14) of the bio-FET, and
the calibration signal (21) is generated based on low-frequency changes and/or a drift of the bias current (14) of the bio-FET (11),
wherein the readout circuit (15) comprises a filter circuit (24, 25, 26);
the filter circuit (24, 25, 26) acts as a low-pass filter for a calibration signal path of the readout circuit (15), to prevent that high-frequency changes of the bias current (14) of the bio-FET (11) are used for generating the calibration signal (21); and
the filter circuit (24, 25, 26) acts as a high-pass filter for a readout signal path of the readout circuit (15), to prevent that low-frequency changes and/or a drift of the bias current (14) of the bio-FET (11) are used for generating the readout signal (16).

## Patentansprüche

1. Biosensorvorrichtung (10), die Folgendes umfasst:
einen oder mehrere Biosensor-Feldeffekttransistoren, Bio-FETs (11); und
einem oder mehrere Ausleseschaltkreise (15), wobei jeder Ausleseschaltkreis (15) mit einem Source-Knoten (13) eines Bio-FET (11) der Bio-FETs (11) verbunden ist und zu Folgendem konfiguriert ist:
Erzeugen eines Auslesesignals (16) basierend auf einem Bias-Strom (14) des Bio-FET (11);
Erzeugen eines Kalibrierungssignals (21) basierend auf einer Stromdifferenz zwischen dem Bias-Strom (14) des Bio-FET (11) und einem Referenzstrom (22); und
Steuern (17) eines Bias des Source-Knotens (13) des Bio-FET (11) gemäß dem Kalibrierungssignal (21), **dadurch gekennzeichnet, dass**
das Auslesesignal (16) basierend auf Hochfrequenzänderungen des Bias-Stroms (14) des Bio-FET erzeugt wird; und
das Kalibrierungssignal (21) basierend auf Niederfrequenzänderungen und/oder einem Drift des Bias-Stroms (14) des Bio-FET (11) erzeugt wird,
wobei der Ausleseschaltkreis (15) einen Filterschaltkreis (24, 25, 26) umfasst;
der Filterschaltkreis (24, 25, 26) dazu konfiguriert ist, als ein Tiefpassfilter für einen Kalibrierungssignalpfad des Ausleseschaltkreises (15) zu wirken, um zu verhindern, dass Hochfrequenzänderungen des Bias-Stroms (14) des Bio-FET (11) zum Erzeugen des Kalibrierungssignals (21) verwendet werden; und
der Filterschaltkreis (24, 25, 26) dazu konfiguriert ist, als ein Hochpassfilter für einen Auslesesignalpfad des Ausleseschaltkreises (15) zu wirken, um zu verhindern, dass Niederfrequenzänderungen und/oder ein Drift des Bias-Stroms (14) des Bio-FET (11) zum Erzeugen des Auslesesignals (16) verwendet werden.

2. Biosensorvorrichtung (10) nach Anspruch 1, wobei
der Ausleseschaltkreis (15) einen Stromkomparatorschaltkreis (23a) umfasst, der dazu konfiguriert ist, das Kalibrierungssignal (21) basierend auf dem Bias-Strom (14) des Bio-FET (11) und dem Referenzstrom (22) zu erzeugen.

3. Biosensorvorrichtung (10) nach Anspruch 2, wobei
der Stromkomparatorschaltkreis (23a) einen ersten Verstärker (26) und ein in Reihe verbundenes Transistorpaar (27b, 27d) umfasst, wobei ein Mittelpunkt des in Reihe verbundenen Transistorpaars (27b, 27d) mit einem invertierenden Eingang des ersten Verstärkers (26) verbunden ist;
eine Referenzspannung (Vref) mit einem nichtinvertierenden Eingang des ersten Verstärkers (26) verbunden ist;
das Transistorpaar (27b, 27d) dazu konfiguriert ist, die Stromdifferenz zwischen dem Bias-Strom (14) des Bio-FET (11) und dem Referenzstrom (22) als eine Spannungsdifferenz im Vergleich zu der Referenzspannung (Vref) an den invertierenden Eingang des ersten Verstärkers (26) bereitzustellen; und
der erste Verstärker (26) dazu konfiguriert ist, das Kalibrierungssignal (21) an seinem Ausgang bereitzustellen.

4. Biosensorvorrichtung (10) nach Anspruch 3, die ferner Folgendes umfasst:
einen Kondensator (25), der mit dem invertierenden Eingang des ersten Verstärkers (26) und dem Ausgang des ersten Verstärkers (26) verbunden ist; und
einen Pseudowiderstand (24), der zwischen dem Mittelpunkt des in Reihe verbundenen Transistorpaars (27b, 27d) und
dem invertierenden Eingang des ersten Verstärkers (26) verbunden ist;
wobei der Kondensator (25), der Pseudowiderstand (24) und der erste Verstärker (26) den Filterschaltkreis (24, 25, 26) bilden.

5. Biosensorvorrichtung (10) nach Anspruch 3 oder 4, wobei
der Ausleseschaltkreis (15) einen ersten Stromspiegelschaltkreis umfasst, der einen ersten Transistor (27a) und einen zweiten Transistor (27b) beinhaltet und dazu konfiguriert ist, den Referenzstrom (22) von dem ersten Transistor (27a) zu dem zweiten Transistor (27b) zu spiegeln;
der Ausleseschaltkreis (15) einen zweiten Stromspiegelschaltkreis umfasst, der einen dritten Transistor (27c) und einen vierten Transistor (27d) beinhaltet und dazu konfiguriert ist, den Bias-Strom (14) des Bio-FET (11) von dem dritten Transistor (27c) zu dem vierten Transistor (27d) zu spiegeln; und
das in Reihe verbundene Transistorpaar (27b, 27d) durch den zweiten Transistor (27b) und den vierten Transistor (27d) gebildet wird.

6. Biosensorvorrichtung (10) nach Anspruch 5, wobei
der Ausleseschaltkreis (15) ferner einen Ausgangstransistor (28) umfasst;
der dritte Transistor (27c) und der vierte Transistor (27d) mit dem Ausgangstransistor (28) verbunden sind; und der Ausgangstransistor (28) dazu konfiguriert ist, das Auslesesignal (16) basierend auf dem Bias-Strom (14) des Bio-FET (11) zu erzeugen.

7. Biosensorvorrichtung (10) nach einem der Ansprüche 2 bis 6, wobei
der Ausleseschaltkreis (15) ferner einen Steuerschaltkreis (23b) umfasst, der dazu konfiguriert ist, den Bias des Source-Knotens (13) des Bio-FET (11) gemäß dem Kalibrierungssignal (21) zu steuern, das durch den Stromkomparatorschaltkreis (23a) bereitgestellt wird.

8. Biosensorvorrichtung (10) nach Ansprüchen 3 und 7, wobei
der Steuerschaltkreis (23b) einen zweiten Verstärker (29) umfasst;
der Ausgang des ersten Verstärkers (26) mit einem nichtinvertierenden Eingang des zweiten Verstärkers (29) verbunden ist; und
der Source-Knoten (13) des Bio-FET (11) mit einem invertierenden Eingang des zweiten Verstärkers (29) verbunden ist.

9. Biosensorvorrichtung (10) nach Anspruch 8, wobei
der Ausleseschaltkreis (15) ferner einen Eingangstransistor (28a) umfasst;
der Eingangstransistor (28a) zwischen dem Source-Knoten (13) des Bio-FET (11) und dem dritten Transistor (27c) des zweiten Stromspiegelschaltkreises verbunden ist; und das Gate des Eingangstransistors mit einem Ausgang des zweiten Verstärkers (29) verbunden ist.

10. Biosensorvorrichtung (10) nach einem der Ansprüche 1 bis 9, wobei
der eine oder die mehreren Bio-FETs (11) jeweils ein Flüssigkeit-Gate umfassen.

11. Biosensorvorrichtung (10) nach einem der Ansprüche 1 bis 10, wobei
der eine oder die mehreren Bio-FETs (11) einen oder mehrere Nanoporen-FETs umfassen.

12. Biosensorvorrichtung (10) nach einem der Ansprüche 1 bis 11, wobei die Biosensorvorrichtung (10) Folgendes umfasst:
ein Array von Bio-FETs (11) und ein Array von Ausleseschaltkreisen (15), die mit dem Array von Bio-FETs (11) verbunden sind.

13. Biosensorvorrichtung (10) nach Anspruch 12, wobei das Array von Ausleseschaltkreisen (15) eine oder mehrere Gruppen von Ausleseschaltkreisen (15) umfasst; und
jede Gruppe von Ausleseschaltkreisen (15) mit einem oder mehreren Multiplexern (51) verbunden ist.

14. Verfahren zum Kalibrieren einer Biosensorvorrichtung (10), welches Folgendes umfasst:
einen oder mehrere Biosensor-Feldeffekttransistoren, Bio-FETs (11); und
einem oder mehrere Ausleseschaltkreise (15), wobei jeder Ausleseschaltkreis (15) mit einem Source-Knoten (13) eines Bio-FET (11) der Bio-FETs (11) verbunden ist, und wobei das Verfahren Folgendes umfasst:
Erzeugen, mit dem Ausleseschaltkreis (15), eines Auslesesignals (16) basierend auf einem Bias-Strom (14) des Bio-FET (11);
Erzeugen eines Kalibrierungssignals (21) basierend auf einer Stromdifferenz zwischen dem Bias-Strom (14) des Bio-FET (11) und einem Referenzstrom (22); und
Steuern (17) eines Bias des Source-Knotens (13) des Bio-FET (11) gemäß dem Kalibrierungssignal (21); **dadurch gekennzeichnet, dass**
das Auslesesignal (16) basierend auf Hochfrequenzänderungen des Bias-Stroms (14) des Bio-FET erzeugt wird, und
das Kalibrierungssignal (21) basierend auf Niederfrequenzänderungen und/oder einem Drift des Bias-Stroms (14) des Bio-FET (11) erzeugt wird,
wobei der Ausleseschaltkreis (15) einen Filterschaltkreis (24, 25, 26) umfasst;
der Filterschaltkreis (24, 25, 26) als ein Tiefpassfilter für einen Kalibrierungssignalpfad des Ausleseschaltkreises (15) wirkt, um zu verhindern, dass Hochfrequenzänderungen des Bias-Stroms (14) des Bio-FET (11) zum Erzeugen des Kalibrierungssignals (21) verwendet werden; und
der Filterschaltkreis (24, 25, 26) als ein Hochpassfilter für einen Auslesesignalpfad des Ausleseschaltkreises (15) zu wirkt, um zu verhindern, dass Niederfrequenzänderungen und/oder ein Drift des Bias-Stroms (14) des Bio-FET (11) zum Erzeugen des Auslesesignals (16) verwendet werden.

## Revendications

1. Dispositif biocapteur (10) comprenant un ou plusieurs transistors à effet de champ de biocapteur, des bio-FET (11) ; et
un ou plusieurs circuits de lecture (15), chaque circuit de lecture (15) étant connecté à un nœud source (13) d'un bio-FET (11) des bio-FET (11) et étant configuré pour générer un signal de lecture (16) sur la base d'un courant de polarisation (14) du bio-FET (11) ;
générer un signal d'étalonnage (21) sur la base d'une différence de courant entre le courant de polarisation (14) du bio-FET (11) et un courant de référence (22), et
commander (17) une polarisation du nœud source (13) du bio-FET (11) en fonction du signal d'étalonnage (21), **caractérisé en ce que**
le signal de lecture (16) est généré sur la base de changements à haute fréquence du courant de polarisation (14) du bio-FET ; et
le signal d'étalonnage (21) est généré sur la base de changements basse fréquence et/ou d'une dérive du courant de polarisation (14) du bio-FET (11),
le circuit de lecture (15) comprenant un circuit de filtrage (24, 25, 26) ;
le circuit de filtrage (24, 25, 26) étant configuré pour agir comme un filtre passe-bas pour un trajet de signal d'étalonnage du circuit de lecture (15), afin d'empêcher que des changements à haute fréquence du courant de polarisation (14) du bio-FET (11) ne soient utilisés pour générer le signal d'étalonnage (21) ; et
le circuit de filtrage (24, 25, 26) étant configuré pour agir comme un filtre passe-haut pour un trajet de signal de lecture du circuit de lecture (15), afin d'empêcher que des changements basse fréquence et/ou une dérive du courant de polarisation (14) du bio-FET (11) ne soient utilisés pour générer le signal de lecture (16).

2. Dispositif biocapteur (10) selon la revendication 1,
le circuit de lecture (15) comprenant un circuit comparateur de courant (23a) configuré pour générer le signal d'étalonnage (21) sur la base du courant de polarisation (14) du bio-FET (11) et du courant de référence (22).

3. Dispositif biocapteur (10) selon la revendication 2,
le circuit comparateur de courant (23a) comprenant un premier amplificateur (26) et une paire de transistors connectés en série (27b, 27d), un point milieu de la paire de transistors connectés en série (27b, 27d) étant connecté à une entrée inverseuse du premier amplificateur (26) ;
une tension de référence (Vref) étant connectée sur une entrée non-inverseuse du premier amplificateur (26) ; la paire de transistors (27b, 27d) étant configurée pour fournir la différence de courant entre le courant de polarisation (14) du bio-FET (11) et le courant de référence (22) sous forme de différence de tension comparée à la tension de référence (Vref) à l'entrée inverseuse du premier amplificateur (26) ; et
le premier amplificateur (26) étant configuré pour fournir le signal d'étalonnage (21) à sa sortie.

4. Dispositif biocapteur (10) selon la revendication 3, comprenant en outre
un condensateur (25) connecté à l'entrée inverseuse du premier amplificateur (26) et à la sortie du premier amplificateur (26) ; et
une pseudo-résistance (24) connectée entre le point milieu de la paire de transistors connectés en série (27b, 27d) et l'entrée inverseuse du premier amplificateur (26) ;
le condensateur (25), la pseudo-résistance (24), et le premier amplificateur (26) formant le circuit de filtrage (24, 25, 26).

5. Dispositif biocapteur (10) selon la revendication 3 ou la revendication 4,
le circuit de lecture (15) comprenant un premier circuit miroir de courant qui comporte un premier transistor (27a) et un deuxième transistor (27b) et est configuré pour mettre en miroir le courant de référence (22) du premier transistor (27a) au deuxième transistor (27b) ;
le circuit de lecture (15) comprenant un deuxième circuit miroir de courant, qui comprend un troisième transistor (27c) et un quatrième transistor (27d) et est configuré pour mettre en miroir le courant de polarisation (14) du bio-FET (11) du troisième transistor (27c) vers le quatrième transistor (27d) ; et la paire de transistors connectés en série (27b, 27d) étant formée par le deuxième transistor (27b) et le quatrième transistor (27d).

6. Dispositif biocapteur (10) selon la revendication 5,
le circuit de lecture (15) comprenant en outre un transistor de sortie (28) ;
le troisième transistor (27c) et le quatrième transistor (27d) étant connectés au transistor de sortie (28) ; et
le transistor de sortie (28) étant configuré pour générer le signal de lecture (16) sur la base du courant de polarisation (14) du bio-FET (11).

7. Dispositif biocapteur (10) selon l'une des revendications 2 à 6,
le circuit de lecture (15) comprenant en outre un circuit de commande (23b) configuré pour commander la polarisation du nœud source (13) du bio-FET (11) en fonction du signal d'étalonnage (21) fourni par le circuit comparateur de courant (23a).

8. Dispositif biocapteur (10) selon la revendication 3 ou la revendication 7,
le circuit de commande (23b) comprenant un deuxième amplificateur (29) ;
la sortie du premier amplificateur (26) étant connectée à une entrée non-inverseuse du deuxième amplificateur (29) ; et
le nœud source (13) du bio-FET (11) étant connecté à une entrée inverseuse du deuxième amplificateur (29).

9. Dispositif biocapteur (10) selon la revendication 8,
le circuit de lecture (15) comprenant en outre un transistor d'entrée (28a) ;
le transistor d'entrée (28a) étant connecté entre le nœud source (13) du bio-FET (11) et le troisième transistor (27c) du deuxième circuit miroir de courant ; et
la grille du transistor d'entrée étant connectée à une sortie du deuxième amplificateur (29).

10. Dispositif biocapteur (10) selon l'une des revendications 1 à 9,
le ou les bio-FET (11) comprenant chacun une grille liquide.

11. Dispositif biocapteur (10) selon l'une des revendications 1 à 10,
le ou les bio-FET (11) comprenant un ou plusieurs FET nanopores.

12. Dispositif biocapteur (10) selon l'une des revendications 1 à 11, le dispositif biocapteur (10) comprenant
un réseau de bio-FET (11) et un réseau de circuits de lecture (15) connectés au réseau de bio-FET (11).

13. Dispositif biocapteur (10) selon la revendication 12,
le réseau de circuits de lecture (15) comprenant un ou plusieurs groupes de circuits de lecture (15) ; et
chaque groupe de circuits de lecture (15) étant connecté à un ou plusieurs multiplexeurs (51).

14. Procédé d'étalonnage d'un dispositif biocapteur (10) comprenant
un ou plusieurs transistors à effet de champ de biocapteur, des bio-FET (11) ; et
un ou plusieurs circuits de lecture (15), chaque circuit de lecture (15) étant connecté à un nœud source (13) d'un bio-FET (11) des bio-FET (11), et le procédé comprenant :
la génération, avec le circuit de lecture (15), d'un signal de lecture (16) sur la base d'un courant de polarisation (14) du bio-FET (11) ;
la génération d'un signal d'étalonnage (21) sur la base d'une différence de courant entre le courant de polarisation (14) du bio-FET (11) et un courant de référence (22), et
la commande (17) d'une polarisation du nœud source (13) du bio-FET (11) en fonction du signal d'étalonnage (21), **caractérisé en ce que**
le signal de lecture (16) est généré sur la base de changements à haute fréquence du courant de polarisation (14) du bio-FET, et
le signal d'étalonnage (21) est généré sur la base de changements basse fréquence et/ou d'une dérive du courant de polarisation (14) du bio-FET (11),
le circuit de lecture (15) comprenant un circuit de filtrage (24, 25, 26) ;
le circuit de filtrage (24, 25, 26) agissant comme un filtre passe-bas pour un trajet de signal d'étalonnage du circuit de lecture (15), afin d'empêcher que des changements à haute fréquence du courant de polarisation (14) du bio-FET (11) ne soient utilisés pour générer le signal d'étalonnage (21) ; et
le circuit de filtrage (24, 25, 26) agissant comme un filtre passe-haut pour un trajet de signal de lecture du circuit de lecture (15), afin d'empêcher que des changements basse fréquence et/ou une dérive du courant de polarisation (14) du bio-FET (11) ne soient utilisés pour générer le signal de lecture (16).
